# EUROPÄISCHE PATENTSCHRIFT

(11) **EP 2 593 069 B1**
(45) Veröffentlichungstag und Bekanntmachung des Hinweises auf die Patenterteilung: **30.05.2018**
(21) Anmeldenummer: 11731344.5
(22) Anmeldetag: 12.07.2011
(51) Int. Cl.: A61K 6/083

(54) **FÜLLSTOFFE FÜR DENTALKOMPOSITE**
FILLERS FOR DENTAL COMPOSITES
MATIÈRES DE CHARGE POUR COMPOSITES DENTAIRES

(30) Priorität: 14.07.2010 EP 10169498
(43) Veröffentlichungstag der Anmeldung: 22.05.2013
(73) Patentinhaber: Quarzwerke GmbH, 50226 Frechen (DE)
(72) Erfinder: KRUBER, Dirk, 53347 Alfter (DE); DOEGE, Thomas, 52249 Eschweiler (DE)
(74) Vertreter: dompatent von Kreisler Selting Werner - Partnerschaft von Patent- und Rechtsanwälten mbB
(86) Internationale Anmeldenummer: PCT/EP2011/061793
(87) Internationale Veröffentlichungsnummer: WO 2012/007440

(56) Entgegenhaltungen:
- EP-A1- 0 747 034
- EP-B1- 1 225 867
- US-A- 3 400 097
- US-B2- 7 294 392

## Beschreibung

Die vorliegende Erfindung betrifft Füllstoffe für Dentalmaterialien.

Im Dentalbereich haben Kompositmaterialien traditionelle Materialien wie Amalgam abgelöst. Einer der wesentlichen Gründe hierfür ist die verbesserte Ästhetik. Kompositmaterialien können in einer Vielzahl von Farben eingefärbt werden, so dass sie dem Farbton der Zähne angeglichen sind.

Kompositmaterialien bestehen aus einem polymerisierbaren Kunstharz und einem Füllstoff. Typischerweise wird das polymerisierbare Harz mit UV-Licht gehärtet. Daher ist es notwendig, dass die Materialien UV-durchlässig sind. Die härtbaren Kunstharze sind in vielen Fällen Acrylate, beispielsweise Bisphenol A-Glycidyl-Methacrylat:
Typische Füllstoffe, die heutzutage in Kompositmaterialien eingesetzt werden, sind Quarze, Gläser und Keramik. In Kompositmaterialien sind die Füllstoffe in einer Menge von typischerweise etwa 70 bis 85 % enthalten, so dass sie die Eigenschaften des Kompositmaterials wesentlich mitbestimmen. Eigenschaften des Füllmaterials, die die Eigenschaften des Kompositmaterials besonders bestimmen, sind die Partikelverteilung und die Partikelform.

In vielen Fällen ist der Füller selbst radioopaque, damit bei Röntgenaufnahmen das Füllmaterial scharf umgrenzt erkannt werden kann. Es gibt jedoch auch Anwendungen, in denen die Radioopapzität nicht von Relevanz ist.

In praktisch allen Kompositmaterialien ist es notwendig, den Füller vorzubehandeln, um eine Verstärkung der Bindung zwischen Füllmaterial und Kunstharz zu erreichen. Ein wesentlicher Aspekt der Qualität eines Komposits ist der Aspekt der Schrumpfung. Ein Komposit, das schrumpft, öffnet zwischen dem Komposit und dem Zahn einen Spalt, der zu weiteren Angriffen am Zahnmaterial führen kann.

Weitere für die praktische Anwendung relevante Eigenschaften sind gute Polierbarkeit, gute Handhabbarkeit, optische Eigenschaften (z.B. UV-Transparenz, geringe Verfärbung), gute Durchhärtung und natürlich auch der Preis.

Das Dokument US 7,294,392 B2 offenbart ein Kompositmaterial, welches aus Feldspatteilchen mit einem mittleren Partikeldurchmesser d50 von 4,5 µm zu einem porösen Grundkörper gesintert wird. Der auf diese Weise gebildete poröse Grundkörper wird in einem nächsten Schritt silanisiert und in einem weiteren Schritt mit einem Polymer ausgefüllt.

In der EP 1 225 867 B1 wird ein Dentalmaterial aus silanisierten Feldspatteilchen mit einem mittleren Partikeldurchmesser ≤ 0,3 µm offenbart.

Aus der EP 0 747 034 A1 sind Pastenopaker bekannt, die unter anderem Feldspatteilchen mit einem mittleren Partikeldurchmesser d50 von 3 bis 6 µm aufweisen.

Das Dokument US 3,400,097 offenbart Fritten aus silanisierten Feldspatteilchen mit Teilchengrößen von 200 bis 325 Mesh zur Herstellung von Porzellanprothesen.

Obwohl eine Vielzahl von verschiedenen Kompositmaterialien und Füllstoffen für Kompositmaterialien existieren, besteht weiterhin Bedarf nach weiteren Füllstoffen mit anderen, bevorzugt zumindest in Teilbereichen besseren Eigenschaften.

Aufgabe der vorliegenden Erfindung war es, solche Füllstoffe bereitzustellen.

Gelöst wird die Aufgabe durch einen pulverförmigen Füllstoff für Dentalmaterialien, enthaltend Teilchen aus Feldspat oder Feldspatderivaten mit einem mittleren Partikeldurchmesser (d50) von 0,5 bis 5 µm, wobei die Teilchen eine Beschichtung mit einer Siliciumverbindung, die reaktive Gruppen enthält, aufweisen, wobei die reaktiven Gruppen polymerisierbare Gruppen enthalten und wobei die Dentalmaterialien Kompositmaterialien sind.

Erfindungsgemäß besteht der pulverförmige Füllstoff also aus Feldspat oder Feldspatderivaten. Feldspatderivate sind insbesondere Materialien, die an Siliciumdioxid verarmt sind, sogenannte Foide bzw. Feldspatoide.

Die erfindungsgemäßen Teilchen weisen einen mittleren Partikeldurchmesser von 0,5 bis 5 µm auf. Der mittlere Partikeldurchmesser wird als d50 bezeichnet. Dies bedeutet, dass aus einer Partikelmischung 50 % (nach Gewicht) der Partikel ein Sieb des entsprechenden Durchmessers passieren können, während 50 % zurückgehalten werden.

Die erfindungsgemäßen Feldspatteilchen oder Feldspatderivatteilchen weisen eine Beschichtung mit einer Siliciumverbindung auf, die reaktive Gruppen enthält. Die Beschichtung muss auf der einen Seite in der Lage sein, mit dem Füllstoff zu reagieren, auf der anderen Seite müssen reaktive Gruppen verbleiben. Solche Reagenzien werden auch bei anderen Füllstoffen auf Basis von Quarz oder Gläsern eingesetzt.

Die Reagenzien weisen auf der einen Seite eine modifizierte Siliciumverbindung auf, die in der Lage ist, mit dem Feldspat eine Reaktion einzugehen, beispielsweise eine Trimethoxysilangruppe.

Weiterhin enthält das Produkt bevorzugt eine polymerisierbare Gruppe, beispielsweise ein Epoxid, ein Acrylat oder Methacrylat oder eine Vinylgruppe, die in der Lage ist, mit einem Kunstharz zu polymerisieren.

Reagenzien hierfür sind dem Fachmann bekannt. Typische Reagenzien sind beispielsweise γ-Methacryloxypropyltrimethoxysilan.

In einigen Ausführungsformen ist es sinnvoll, verschiedene Modifizierungsreagenzien zu mischen, um die Füllstoffe zu beschichten.

Als Feldspate haben sich Mitglieder der Gruppe Kalknatronfeldspate oder Alkalifeldspate als besonders geeignet erwiesen. Geeignete Mineralien sind insbesondere Perthit, Albit, Oligoklas, Andesin, Labradorit, Bytownit, Anorthit sowie SiO₂ ärmere Feldspatderivate wie Nephelin und Mischungen davon.

Bevorzugt liegt der mittlere Partikeldurchmesser des Feldspats im Bereich von 0,5 bis 3,5 µm, bevorzugt in Bereich von 0,8 bis 1,5 µm.

Vorzugsweise ist der Feldspat oder das Feldspatderivat lichtdurchlässig, um beispielsweise eine photoinitiierte Polymerisation in einem System, in dem der Feldspat oder das Feldspatderivat als Füllstoff verwendet wird, zu ermöglichen.

Bevorzugterweise ist das Licht ein blaues Licht und weist einen Wellenlängenbereich von 400 bis 520 nm auf. Geeignete Lichtquellen sind hierbei Halogenlampen oder Leuchtdioden, sogenannte LEDs.

In einer Ausführungsform weist der Füllstoff eine zumindest bimodale Partikeldurchmesserverteilung auf, d.h. in der Korngrößenverteilung existieren zwei oder mehr Maxima. In solchen Fällen liegen ein Maximum bevorzugt im Bereich von 0,5 bis 1 µm und das andere Maximum im Bereich von 1 bis 3,5 µm. Solche bi- oder höher modalen Verteilungen werden beispielsweise hergestellt durch das getrennte Mahlen und Sieben von Materialien auf zwei Korngrößenverteilungen der gewünschten Größe und anschließendes Vermischen.

Dabei kann das Vermischen von gleichen Gewichtsmengen dieser Korngruppen oder mit unterschiedlichen Mengen erfolgen. Beispielsweise könnte die eine Korngrößenverteilung in einer Menge von 30 bis 70 Gew.-% und die andere in einem Bereich von 70 bis 30 Gew.-% eingesetzt werden.

Um Feldspat auf die geeignete Größe zu mahlen ist es in vielen Fällen sinnvoll, eine Zweistufenvermahlung einzusetzen.

Eine besonders bevorzugte Variante für die erste Vermahlung ist die sogenannte autogene Luftstrahlvermahlung. Hierbei werden Partikel beschleunigt und aufeinander gestoßen, so dass sie dabei vermahlen werden. Hierdurch können Feldspate in einen Korngrößenbereich bis hinunter zu etwa 1,5 µm vermahlen werden.

Für die weitere Vermahlung eignen sich insbesondere Nassmahlverfahren, beispielsweise unter Einsatz von Rührwerkskugelmühlen. Nach den Nassmahlverfahren wird der Füllstoff getrocknet.

In einer besonders bevorzugten Ausführungsform werden zum Vermahlen Mahlkörper eingesetzt, deren Brechungsindex nahe am Brechungsindex des eingesetzten Feldspats oder Feldspatderivats liegt. Bevorzugt liegt der Unterschied der Brechungsindizes der eingesetzten Mahlkörper und des Feldspates bei nicht mehr als 0,005 auseinander. Beispielsweise können bei einer Rührwerkskugelmühle als Mahlkörper Glaskugeln des entsprechenden Brechungsindex eingesetzt werden. Bevorzugt enthält das erhaltene gemahlene Material weniger als 0,5 Gew.-% einer Verunreinigung durch Mahlkörperabrieb; dies kann z.B. durch Röntgenfluoreszenzanalyse bestimmt werden.

Nach dem Trocknen wird der Füllstoff in bekannter Weise silanisiert. Die Verfahren unterscheiden sich nicht grundsätzlich von der Silanisierung anderer Träger.

In einer besonders bevorzugten Ausführungsform wird ein Dentalkompositmaterial, enthaltend 60 bis 90 Gew.-% des pulverförmigen Füllstoffs und 10 bis 40 Gew.-% eines polymerisierbaren Harzes, gebildet.

Bevorzugt wird das Dentalkompositmaterial mittels Licht polymerisiert bzw. ausgehärtet. Üblicherweise wird ein Licht mit einem Wellenlängenbereich von 400 bis 520 nm verwendet.

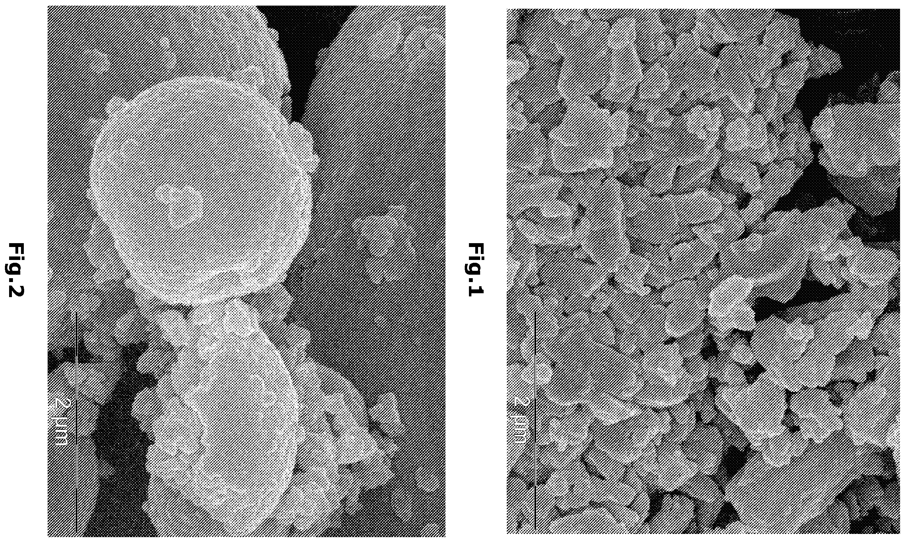

Figur 1 zeigt einen erfindungsgemäßen Füllstoff in einer Korngröße von 0,3 µm.
Figur 2 zeigt den erfindungsgemäßen Füllstoff einer Korngröße von 3,5 µm.

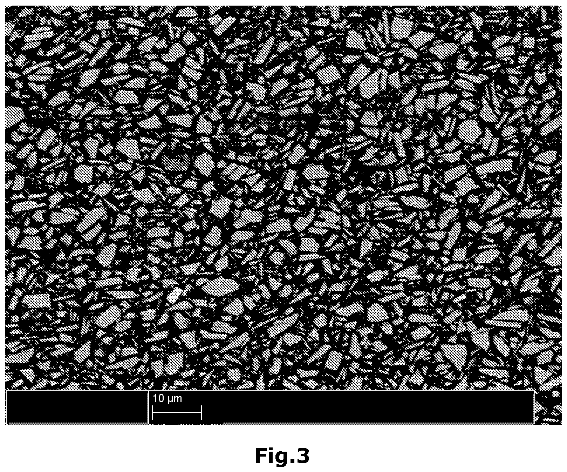

Figur 3 zeigt ein Kompositmaterial unter Verwendung des erfindungsgemäßen Materials nach dem Aushärten und Polieren der Oberfläche. Es handelt sich um rasterelektronenmikroskopische Aufnahmen.

### Bespiel 1

Ein polymerisierbares Kunstharz enthaltend Bis-GMA (2,2-bis[4-(2-hydroxy-3-methylacryloxypropoxy)-phenyl]propane zusammen mit TEGDMA (2-methyl-2-propenoic acid) wurde hergestellt. Als Photoinitiator wurde Camphorchinon und 2-dimethylaminoethylmethacrylat eingesetzt.

Als Feldspat diente ein Feldspat, der mit γ-Methacryloxypropyltrimethoxysilanbeschichtet war. Das Vermischen des polymerisierbaren Harzes und des Füllers erfolgte durch Mischen per Hand. Es wurden folgende Feldspatkorngrößen verwendet:
a) Korngröße 0,3 µm (Vergleichsbeispiel)
b) Korngröße 0,8 µm
c) Korngröße 3,5 µm
d) Mischung der Füller 0,8 µm und 3,5 µm in den Gewichtsverhältnissen 40:60

Als Vergleichsbeispiele wurden eingesetzt:
V1: Bariumglas Korngröße 0,7 µm (GM 39923 der Firma Schott)
V2: Bariumglas Korngröße 1,0 µm (GM 27884 der Firma Schott)

### Beispiel 2

Es wurden folgende Kompositmaterialien hergestellt:
Füllstoff a) 60 %, Kunstharz 40 %
Füllstoff b) 67 %, Kunstharz 33 %
Füllstoff c) 73 %, Kunstharz 27 %
Füllstoff d) 74 %, Kunstharz 36 %
Füllstoff V1 68 %, Kunstharz 32 %
Füllstoff V2 72 %, Kunstharz 28 %

Aushärtung erfolgte bei einem 6 mm Prüfkörper mit einer Dentacolor XS (Heraeus Kulzer) für 180 s.

Anschließend wurden verschiedene Eigenschaften der Materialien untersucht. Die Ergebnisse sind in der folgenden Tabelle dargestellt.

| | Biegefestigkeit [MPa] | Scherfestigkeit [MPa] | Vickers-Härte [HV 5-20] | Rauigkeit 1) Ra in [µm] |
|---|---|---|---|---|
| V1 0.7 µm | 115,6 | 22,6 | 47,0 | n.b. |
| V2 1.0 µm | 145,0 | 31,3 | 54,4 | n.b. |
| (a) 0.3 µm (*) | 144,0 | 19,7 | 48,5 | 0,05 |
| (b) 0.8 µm | 212,0 | 29,7 | 53,9 | 0,05 |
| (c) 3.5 µm | 205,0 | 28,2 | 51,3 | 0,05 |
| (d) bimodal | 203,0 | 31,3 | 47,6 | 0,05 |

| | | | | |
|---|---|---|---|---|
| (*) Vergleichsbeispiel ¹⁾ Nach Schleifen mit: 1. Stufe: Aufrauen der Oberfläche mit Hartmetallfräser 2. Stufe: CompoMaster Coarse (Fa. Shofu) 3. Stufe: CompoMaster (Fa. Shofu) 4. Stufe: DirectDia Paste; Super Snap Buff Disk (Fa. Shofu) n.b. - nicht bestimmt | | | | |

Verglichen mit üblichen Dentalfüllmaterialien auf Basis von Strontium- oder Bariumgläsern zeigten die erfindungsgemäßen Füllstoffe gleiche oder teilweise bessere mechanische Eigenschaften. In den Kompositsystemen wurden mit den erfindungsgemäßen Füllstoffen sehr gute Durchhärtungsergebnisse erreicht.

Die lineare Schrumpfung betrug 1,4 bis 1,7 % und war damit besser als im Stand der Technik. Es konnten hohe Füllstoffgehalte erreicht werden und es ergab sich trotzdem eine gute Verarbeitbarkeit der erfindungsgemäßen Komposite. Die Materialien waren hochtransparent, so dass sie keine Veränderung bei der Färbung bewirkten.

## Patentansprüche

1. Pulverförmiger Füllstoff für Dentalmaterialien, enthaltend Teilchen aus Feldspat oder Feldspatderivaten mit einem mittleren Partikeldurchmesser (d50) von 0,5 bis 5 µm, wobei die Teilchen eine Beschichtung mit einer Siliciumverbindung, die reaktive Gruppen enthält, aufweisen, wobei die reaktiven Gruppen polymerisierbare Gruppen enthalten und wobei die Dentalmaterialien Kompositmaterialien sind.

2. Pulverförmiger Füllstoff nach Anspruch 1, wobei die polymerisierbaren Gruppen Epoxy- oder Vinylgruppen, bevorzugt Methacryl- oder Acrylgruppen enthalten.

3. Pulverförmiger Füllstoff nach einem der Ansprüche 1 oder 2, wobei der Feldspat ausgewählt ist aus der Gruppe der Kalknatronfeldspate oder Alkalifeldspate.

4. Pulverförmiger Füllstoff nach einem der Ansprüche 1 bis 3, wobei der Feldspat ausgewählt ist aus Perthit, Albit, Oligoklas, Andesin, Labradorit, Bytownit, Anorthit sowie SiO₂ ärmere Feldspatderivate wie Nephelin und Mischungen davon.

5. Pulverförmiger Füllstoff nach einem der Ansprüche 1 bis 4, wobei der Feldspat einen mittleren Partikeldurchmesser (d50) von 0,8 bis 1,5 µm aufweist.

6. Pulverförmiger Füllstoff nach einem der Ansprüche 1 bis 5, wobei der Feldspat lichtdurchlässig ist.

7. Pulverförmiger Füllstoff nach einem der Ansprüche 1 bis 6, wobei der Füllstoff eine bimodale Partikeldurchmesser-Verteilung aufweist.

8. Pulverförmiger Füllstoff nach Anspruch 7, wobei ein Maximum der bimodalen Verteilung im Bereich 0,5 bis 1 µm und das andere Maximum im Bereich von 1 bis 3,5 µm liegt.

9. Verfahren zur Herstellung eines pulverförmigen Füllstoffs nach einem der Ansprüche 1 bis 8 mit folgenden Schritten:
- Mahlen von Feldspat
- Silanisieren der Partikel mit einer reaktiven Siliciumverbindung.

10. Dentalkompositmaterial enthaltend
- 60 bis 90 Gew.-% eines pulverförmigen Füllstoffs nach einem der Ansprüche 1 bis 8
- 10 bis 40 Gew.-% eines polymerisierbaren Harzes, wobei das polymerisierbare Harz mit den reaktiven Gruppen reagieren kann.

11. Dentalkompositmaterial nach Anspruch 10, wobei das Dentalkompositmaterial mittels Licht aushärtbar ist.

12. Dentalmaterial enthaltend ausgehärtetes Kompositmaterial nach Anspruch 10 oder 11.

13. Verwendung des pulverförmigen Füllstoffes nach einem der Ansprüche 1 bis 8 als Füllstoff in Dentalmaterialen.

## Claims

1. A powdery filler for dental materials comprising particles of feldspar or feldspar derivatives having a mean particle diameter (d50) of from 0.5 to 5 µm, the particles having a coating with a silicon compound comprising reactive groups, wherein said reactive groups contain polymerizable groups, and wherein said dental materials are composite materials.

2. The powdery filler according to claim 1, wherein said polymerizable groups comprise epoxy or vinyl groups, preferably methacrylic or acrylic groups.

3. The powdery filler according to either of claims 1 or 2, wherein said feldspar is selected from the group of plagioclase feldspars or alkali feldspars.

4. The powdery filler according to any of claims 1 to 3, wherein said feldspar is selected from perthite, albite, oligoclase, andesine, labradorite, bytownite, anorthite as well as SiO₂-deficient feldspar derivatives, such as nepheline, and mixtures thereof.

5. The powdery filler according to any of claims 1 to 4, wherein said feldspar has a mean particle diameter (d50) of from 0.8 to 1.5 µm.

6. The powdery filler according to any of claims 1 to 5, wherein said feldspar is transparent.

7. The powdery filler according to any of claims 1 to 6, wherein said filler has a bimodal particle diameter distribution.

8. The powdery filler according to claim 7, wherein one peak of said bimodal distribution is within a range of from 0.5 to 1 µm, and the other peak is within a range of from 1 to 3.5 µm.

9. A process for preparing a powdery filler according to any of claims 1 to 8, with the following steps:
- grinding feldspar
- silanizing the particles with a reactive silicon compound.

10. A dental composite material comprising
- from 60 to 90% by weight of a powdery filler according to any of claims 1 to 8;
- from 10 to 40% by weight of a polymerizable resin, wherein said polymerizable resin can react with the reactive groups.

11. The dental composite material according to claim 10, wherein said dental composite material can be cured by means of light.

12. A dental material comprising a cured composite material according to claim 10 or 11.

13. Use of the powdery filler according to any of claims 1 to 8 as a filler in dental materials.

## Revendications

1. Masse de remplissage pulvérulente pour matériaux dentaires, contenant des particules de feldspath ou de dérivés de feldspath avec un diamètre de particules moyen (d50) de 0,5 à 5 µm, les particules présentant un revêtement avec un composé de silicium qui contient des groupes réactifs, les groupes réactifs contenant des groupes polymérisables, et les matériaux dentaires étant des matériaux composites.

2. Masse de remplissage pulvérulente selon la revendication 1, les groupes polymérisables contenant des groupes époxydes ou vinyles, de préférence des groupes méthacryles ou acryles.

3. Masse de remplissage pulvérulente selon l'une des revendications 1 ou 2, le feldspath étant sélectionnés à partir du groupe des feldspaths sodocalciques ou des feldspaths alcalins.

4. Masse de remplissage pulvérulente selon l'une des revendications 1 à 3, le feldspath étant sélectionné parmi la perthite, l'albite, l'oligoclase, l'andésine, la labradorite, la bytownite, l'anorthite ainsi que des dérivés de feldspath plus pauvres en SiO₂ tels que la néphéline et leurs mélanges.

5. Masse de remplissage pulvérulente selon l'une des revendications 1 à 4, le feldspath présentant un diamètre de particules moyen (d50) de 0,8 à 1,5 µm.

6. Masse de remplissage pulvérulente selon l'une des revendications 1 à 5, le feldspath étant translucide.

7. Masse de remplissage pulvérulente selon l'une des revendications 1 à 6, la matière de remplissage présentant une distribution bimodale du diamètre de particules.

8. Masse de remplissage pulvérulente selon la revendication 7, un maximum de la distribution bimodale se situant dans la plage de 0,5 à 1 µm, et l'autre maximum se situant dans la plage de 1 à 3,5 µm.

9. Procédé de fabrication d'une masse de remplissage pulvérulente selon l'une des revendications 1 à 8, avec les étapes suivantes :
- broyage du feldspath
- silanisation des particules avec un composé de silicium réactif.

10. Matériau composite dentaire, contenant
- 60 à 90 % en poids d'une masse de remplissage pulvérulente selon l'une des revendications 1 à 8,
- 10 à 40 % en poids d'une résine polymérisable, la résine polymérisable pouvant réagir avec les groupes réactifs.

11. Matériau composite dentaire selon la revendication 10, le matériau composite dentaire pouvant être durci au moyen de la lumière.

12. Matériau dentaire, contenant du matériau composite durci selon la revendication 10 ou 11.

13. Utilisation de la masse de remplissage pulvérulente selon l'une des revendications 1 à 8 en tant que masse de remplissage dans des matériaux dentaires.
